# EUROPÄISCHE PATENTSCHRIFT

(11) **EP 0 387 819 B1**
(45) Veröffentlichungstag und Bekanntmachung des Hinweises auf die Patenterteilung: **18.05.1994**
(21) Anmeldenummer: 90104774.6
(22) Anmeldetag: 14.03.1990
(51) Int. Cl.: A01K 31/17, F24F 7/00

(54) **Antrieb für Luftströmungseinrichtung einer Käfigbatterie für die Geflügelhaltung**
Drive for an aeration device for a battery cage for poultry
Commande pour mécanisme d'aération d'une batterie de cages d'élevage pour volaille

(30) Priorität: 15.03.1989 DE 3908336
(43) Veröffentlichungstag der Anmeldung: 19.09.1990
(73) Patentinhaber: SALMET GESELLSCHAFT MIT BESCHRÄNKTER HAFTUNG & Co. KOMMANDITGESELLSCHAFT, D-74930 Ittlingen (DE)
(72) Erfinder: Lackner, Karl Horst, D-6921 Ittlingen (DE)
(74) Vertreter: Utermann, Gerd, Dipl.-Ing.

(56) Entgegenhaltungen:
- DE-A- 3 715 735
- US-A- 3 040 976

## Beschreibung

Die Erfindung betrifft einen Antrieb für die Luftströmungseinrichtungen einer Käfigbatterie für die Geflügelhaltung mit wenigstens zwei benachbart angeordneten, horizontalen Käfigreihen, unter denen sich jeweils eine Kotaufnahme, ein Kotband, erstreckt, der zum Trocknen des aufgefangenen Kots eine Luftströmungseinrichtung zugeordnet ist, mittels der über die Kotaufnahme hinwegstreichende Luftströme erzeugt werden, wobei die Luftströmungseinrichtung mehrere, über der Kotaufnahme in Längsabständen verteilt angeordnete und auf Querachsen gelagerte Luftfächerklappen aufweist, denen zumindest gruppenweise zusammengefaßte Schwenkantriebe zum Verschwenken der Luftfächerklappen zugeordnet sind, wobei die Luftfächerklappen in der Mittelstellung der Schwenkantriebe im wesentlichen senkrecht ausgerichtet sind und wobei der Schwenkantrieb sich in Längsrichtung durch die Käfigbatterie erstreckende, in geringem Abstand von den Querachsen der Luftfächerklappen, an diesen angreifende Zugorgane aufweist, die jeweils phasenversetzt zueinander hin- und hergehend und zumindest gruppenweise gemeinsam antreibbar sind.

Zur sinnvollen Legehennenhaltung ist Kottrocknung zweckmäßig. Diese wurde früher mit Gebläsen bewirkt. Das ist sowohl konstruktiv als auch bezüglich des Energieeinsatzes aufwendig. Außerdem ergeben sich Schwierigkeiten mit der angesaugten Frischluft, die zu Kondensat führen kann.

Eine wesentliche Verbesserung bietet die aus der DE 37 15 735 A1 bekannte Käfigbatterie für die Geflügelhaltung, die mit Fächerklappen und Schwenkantrieben arbeitet, deren Merkmale vorstehend aufgeführt sind.

Die dazu verwendeten Schwenkantriebe wurden mit Zug- und Druckstangen bzw. mit zwei gegenläufig arbeitenden reinen Zugstangen gebildet. Diese Antriebe haben hohe Massenkräfte und vor allem ergeben sich bei den großen Längen von Käfigbatterien ganz erhebliche Längungen der Zugstangen während des Betriebs, die zu ungleichmäßigem Lauf und erheblichen Schwingungen des ganzen Systems führen.

Der Erfindung liegt die Aufgabe zugrunde, einen Antrieb mit guten Laufeigenschaften, einfachem Aufbau und geringem Energiebedarf vorzusehen. Das wird erreicht, indem an jedem Zugorgan mindestens eine entgegen der Richtung der Antriebskraft wirkende Feder angeordnet ist. Diese Federn sind so auszulegen, daß die Zugorgane stets - auch bei der Rückwärtsbewegung unter einer geeigneten Zugspannung stehen, die eine gleichmäßige Längung des Zugorganes bewirkt und somit schon von vornherein das Einstellen der Angriffselemente der Fächerklappen an den Zugorganen auf einen geeigneten, die Vorspannung berücksichtigenden Abstand ermöglicht. Da diese Vorspannung im wesentlichen gleich bleibt und man nunmehr Zugseile verwenden kann, fallen Ungleichmäßigkeiten durch geringfügige zusätzliche Längungen des Zugorganes nicht mehr ins Gewicht. Zugseile können im Betrieb ohne Beeinträchtigung der Laufeigenschaften zeitweise geringfügig gestaucht werden oder von ihrer Zugspannung entlastet werden. Je Käfigreihe ist nur noch ein Zugorgan anzuordnen, um einen gut arbeitenden Antrieb zu erreichen.

Da mehrere Zugseile verschiedener Käfiggruppen phasenverschoben gekoppelt sind, haben die Federn auf den gesamten Energieeinsatz keinen nennenswerten Einfluß, weil das Zusammendrücken einer Feder oder Federngruppe durch das Freigeben der Federkraft anderer Federn kompensiert wird. Insgesamt kann man mit einem solchen, sinnvoll gestaltetem Antrieb etwa 2/3 des Energieeinsatzes für Gebläsetrocknungen einsparen und erreicht bei günstigerem Käfigklima noch eine bessere Trocknung.

Zweckmäßigerweise wird am der Antriebseite abgewandten Ende jeder Käfigbatterie eine relativ starke Feder angeordnet, während je nach Länge der Käfigbatterie an entsprechenden Zwischenstellen weitere Federn vorgesehen sind, so daß das Zugseil über die ganze Länge etwa unter gleichen Zugkräften steht und die Federn nicht zu groß dimensioniert zu werden brauchen. Dabei können die Federn als Druckfedern ausgebildet und das Zugseil umgebend angeordnet sein.

Als Antrieb für das Antriebsende des Zugseiles kommen die verschiedensten hin- und hergehenden Antriebe in Betracht, insbesondere kann man Schwinghebel anordnen, die mit Hilfe von Exzentern oder Kurvenscheiben angetrieben sind. Die Antriebe werden zweckmäßig mit Winkeleinstell-Kupplungen ausgestattet, um das ganze System in eine die Federkräfte und -Dimensionen berücksichtigende sinnvolle Phasenlage beim Antrieb bringen zu können. Weitere Einzelheiten, Vorteile, Merkmale und Gesichtspunkte sind auch in den weiteren Ansprüchen und dem nachfolgenden, anhand der Zeichnungen abgehandelten Beschreibungsteil behandelt.

Ein Ausführungsbeispiel der Erfindung wird nachfolgend anhand der Zeichnungen erläutert.

Es zeigen:
- Fig. 1: Die schematische Seitenansicht eines Teiles einer Käfigbatterie;
- Fig. 2: einen schematisierten Horizontalschnitt längs der Linie 2-2 in Fig. 1
und
- Fig. 3: den schematisierten Vertikalschnitt längs der Linie 3-3 in Fig. 1 und 2.

Die Käfigbatterie 10 weist sechs horizontal verlaufende Käfigreihen 11.1 bis 11.3 auf, die in drei Etagen übereinander angeordnet sind. Dabei bilden die in jeder Etage vorhandenen zwei Käfigreihen 11 je eine Doppelkäfigreihe, bei der die einzelnen Käfige 13 paarweise rückseitig aneinander stoßen. Jeder Käfig 13 ist mit einem von seiner Rückseite 14 zur Vorderseite 15 abfallenden Gitterboden 16 versehen, der über die Vorderseite 15 vorspringt und eine Eisammelrinne 17 bildet. Über dieser ist ein Futtertrog 18 vorgesehen, der ebenso wie die Eisammelrinne 17 über die ganze Länge der Käfigreihe 11 durchläuft. Die Käfigbatterie 10 ist über Füße 19 abgestützt, die im Bereich der Käfigvorderseiten 15 jeweils zwischen benachbarten Käfigen der beiden unteren Käfigreihen 11.1 angeordnet sind. Käfigbatterien können auch mehr Etagen als drei und mehr Käfigreihen als sechs haben. Sie sind in der Regel viele Meter lang , oft 50 m und mehr.

Jede Käfigreihe 11 hat als Kotaufnahme ein Kotband 20, das sich im Abstand unterhalb der entgegengesetzt geneigten Gitterböden 16 der beiden Käfige 13 der einzelnen Käfigreihen 11 erstreckt. An seinen beiden Rändern sind an den Käfigvorderseiten 15 in Längsrichtung durch die Käfigbatterie 10 verlaufende, ortsfeste, aufragende Randbegrenzungen 21 angeordnet. Das als endloses Umlaufband ausgebildete Kotband 20 fängt den durch die Gitterböden 16 herabfallenden Kot 22 auf und gestattet es, diesen von Zeit zu Zeit an Ende abzugeben. Das rücklaufende Leertrum 20.1 ist ohne die Umlenkrollen nur schematisch angedeutet.

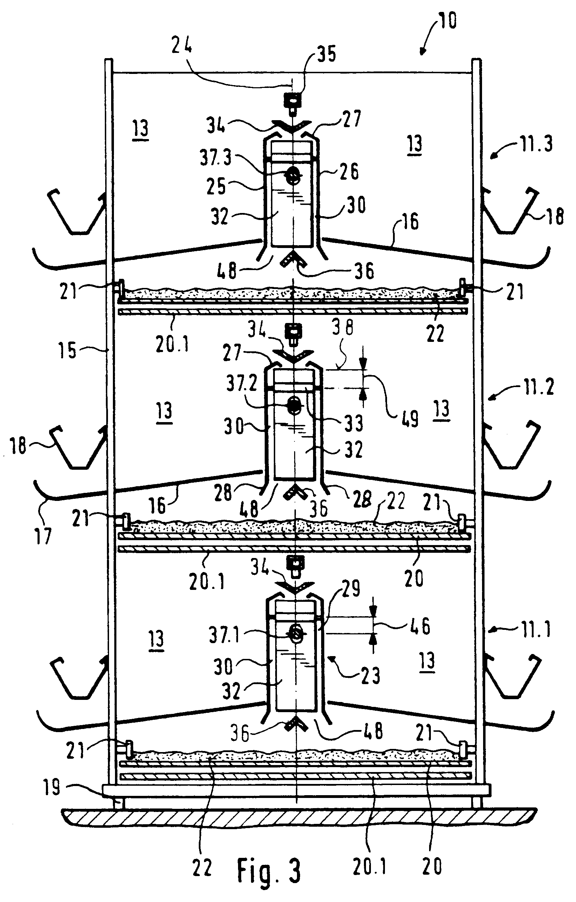

Jede Doppelkäfige aufweisende Käfigreihe 11 ist mit einer Luftströmungseinrichtung 23 versehen, die - wie aus Fig. 3 zu entnehmen - im wesentlichen beiderseits der Längsmittelebene 24 der Käfigbatterie 10 zwischen den einander rückseitig benachbarten Käfigen 13 angeordnet ist. Die Luftströmungseinrichtung 23 weist zwei im wesentlichen senkrecht angeordnete Längswände 25 und 26 auf, die sich auf gegenüberliegenden Seiten der Längsmittelebene 24 parallel über die gesamte Käfigreihe 11 erstrecken. Die oberen Randstreifen 27 der Längswände 25, 26 sind einwärts zur Längsmittelebene 24 hin abgewinkelt, während die unteren Randstreifen 28 der Längswände 25 und 26 nach außen abgewinkelt sind. Die geschlossenen Längswände 25 und 26 bilden jeweils einen Teil der Rückseite 14 der Käfige 13. Sie schließen einen längs verlaufenden Kanal 29 ein, der in einzelne Kammern 30 unterteilt ist, die an ihrer Oberseite zwischen den Randstreifen 27 eine schmale Öffnung aufweisen und nach unten zwischen den Randstreifen 28 offen sind. Die Unterteilung des Kanals 29 in Kammern 30 wird durch Querwände 31 erreicht, die zugleich einander in Längsrichtung benachbarte Käfige 13 voneinander trennen. Dementsprechend ist jedes Käfig-Paar 13-13 mit einer eigenen Kammer 30 versehen, deren Reihe sich in Längsrichtung über die gesamte Käfiglänge erstreckt.

In jeder Kammer 30 ist eine flache Luftfächerklappe 32 mittels einer Querachse 33 gelagert, deren Enden an den Längswänden 25 bzw. 26 abgestützt oder gelagert sind. Die Querachse 33 ist - wie dargestellt - im Bereich des oberen Endes der Luftfächerklappe 32 in einem kleinen Abstand 49 von deren Oberkante 38 angeordnet. Normalerweise hängt daher die Luftfächerklappe 32 infolge ihres Eigengewichtes in einer senkrechten Stellung zwischen den Längswänden 25 und 26, zu denen sie einen geringen Spaltabstand aufweist.

Wie dargestellt, sind die Öffnungen am oberen Ende der Kammern 30 durch eine längs verlaufende Leiste 34 abgedeckt, die als Rinne unter einem Längsrohr 35 verläuft und sich in der Längsmittelebene 24 durch die ganze Käfigreihe 11 erstreckt. Dem offenen unteren Ende der Kammern 30 ist ein längs verlaufender winkelförmiger Luftstromteiler 36 zugeordnet, der sich mit geringem Abstand unterhalb der herabhängenden Luftfächerklappen 32 ebenfalls in der Längsmittelebene 24 über die ganze Käfigreihe 11 erstreckt. Dadurch werden jeweils zwei Luftaustrittsöffnungen 48 geschaffen.

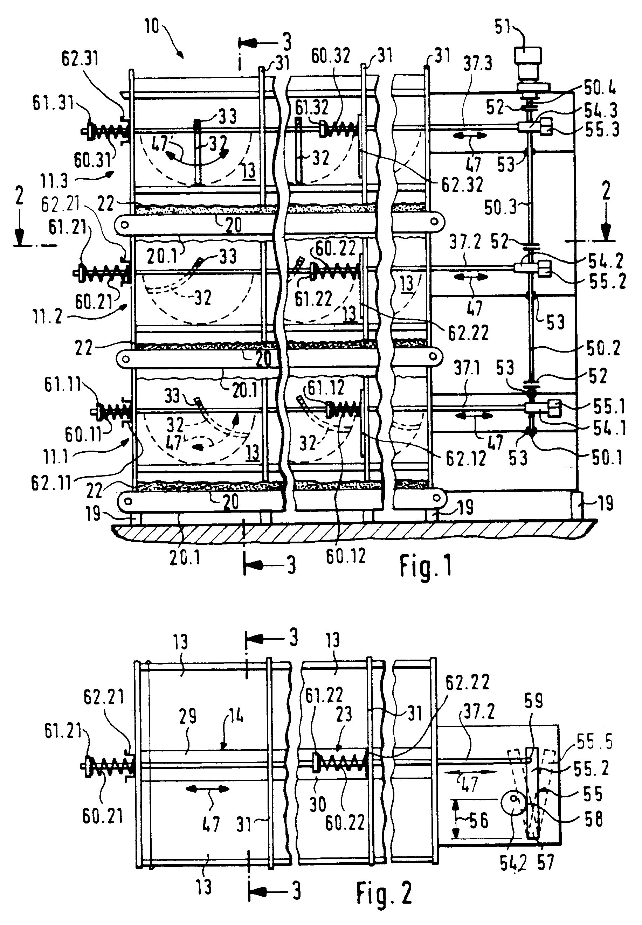

Die Schwenkantriebe zum Verschwenken der Luftfächerklappen 32 in den Kammern 30 sind für jede Käfigreihe 11 mit einem Zugseil 37 als Zugorgan gebildet, das sich im oberen Bereich des betreffenden Kanals 29 in der Längsmittelebene 24 durch die gesamte Käfigreihe 11 erstreckt. Das Zugseil 37 greift jeweils in einem geringen Abstand 46 unter der Querachse 33 jeder Luftfächerklappe 32 der betreffenden Doppelkäfigreihe an der Luftfächerklappe 32 gelenkig an und ist in Richtung seiner Längserstreckung hin und her bewegbar. Das ist ebenso, wie die dadurch erzielte Verschwenkung der Luftflächerklappen 32, in Fig. 1 und 2 durch Doppelpfeile 47 angedeutet. Die in diesem Beispiel ingesamt drei Zugseile 37.1, 37.2, 37.3 werden an einer Stirnseite der Käfigbatterie 10 gemeinsam angetrieben, wie es rechts in Fig. 1 und 2 dargestellt ist. Dafür ist eine gemeinsame Antriebswelle 50 vorgesehen, die in Drehlagern 53 gelagert ist und mit Hilfe eines Getriebemotors 51 angetrieben wird. Die Antriebswelle 50 besteht aus vier Wellenstücken 50.1 bis 50.4, die untereinander durch Kupplungen 52 verbunden sind, so daß die relative Lage der Wellenstücke 50.1 bis 50.4 zueinander einstellbar ist. Auf den drei unteren Wellenstücken 50.1 bis 50.3 ist jeweils eine Exzenterscheibe 54.1 bis 54.3 unverdrehbar befestigt. Wie aus Fig. 1 und 2 ersichtlich, ist jeder Exzenterscheibe je ein Exzenter-Schwinghebel 55 zugeordnet, der in einem Abstand 56 von der Antriebswelle 50 mittels eines Lagers 57 schwenkbar gelagert ist und mit geeigneter Abstütz-Ausbildung mit einem Anlagebereich 58 an der Exzenterscheibe 54.1 bis 54.3 lose von der den Käfigen 13 abgewandten Seite anliegt. Er reicht mit einem längeren Armteil 55.5 bis zu seinem Angriffslager 59, an dem das Ende des Zugseiles 37, vorzugsweise längeneinstellbar abgestützt ist.

Jedes Zugseil 37.1 bis 37.3 bat hei diesem Ausführungsbeispiel zwei Druckfedern 60, die entsprechend den Käfigreihen 11.1, 11.2, 11.3 mit 60.11, 60.12; 60.21, 60.22 und 60.31, 60.32 bezeichnet sind. Die Zugseile erstrecken sich durch die Federn 60. Die Federn 60 sind mit Zugseil-Federlager 61.11 bis 61.32 an den Zugseilen 37.1 bis 37.3 abgestützt.

Das der Exzenterscheibe zugewandte Ende jeder Feder 60 stützt sich an einem Festlager 62.11 bis 62.32 ab, welches mit dem Gestell fest verbunden ist und eine Federkappe sein kann. Die Zugseil-Federlager 61 sind vorzugsweise mit Nachstelleinrichtungen, unter Verwendung von Muttern und Kontermuttern, Keileinrichtungen, geteilten Klemmscheiben oder dgl., ausgestattet.

Wie die Fig. 1 und 2 zeigen, ist am linken Ende der Käfigbatterie 10 jeweils eine größere und stärkere Druckfeder 60.11, 60.21, 60.31 vorgesehen, die die Hauptzugkraft für den größten Teil des Zugseiles 37.1 bis 37.3 aufbringt. Je nach Länge der Käfigbatterie sind an entsprechenden Zwischenstellen weitere Zugkräfte aufbringende Druckfedern, hier im Beispiel die Druckfedern 60.12, 60.22 und 60.32 vorgesehen, um alle Seilabschnitte gleichmäßig und sinnvoll vorgespannt zu halten. Dabei ist die Verteilung der Federn auf die Länge des jeweiligen Zugseiles abzustimmen und die Federkräfte sind entsprechend der Länge der Seilabschnitte und entsprechend der Lage im Seil so zu wählen, daß ein ruhiger Antrieb erfolgt.

Wie die Fig. 1 zeigt, sind die Exzenterscheiben 54.1 bis 54.3 phasenverschoben oder gleichmäßig winkelversetzt unter Winkeln von 120° zueinander auf der Antriebswelle 50 befestigt. Dazu dienen die Kupplungen 52, mit deren Hilfe die einzelnen Wellenstücke 50.1, 50.2, 50.3 gegeneinander verdreht und dann miteinander drehfest verbunden werden können.

Wie Fig. 1 veranschaulicht, befinden sich die oberen Luftfächerklappen 32 der oberen Käfigreihe 11.3 in der mittleren herabhängenden Stellung und der Exzenter-Schwinghebel 55 befindet sich in einer mittleren Auslenkungslage. Die mittlere Käfigreihe 11.2 befindet sich in einem solchen Zustand ihrer Bewegungs-Phase, in der die Luftfächerklappen 32 nach links ausgelenkt sind und den Rückweg gerade begonnen haben. Demgemäß sind die Federn 60.21 und 60.22 gerade am weitesten entspannt und der Exzenter-Schwinghebel 55.2 hat seinen geringsten Abstand zur Antriebswelle 50 und damit seine am nächsten zur Käfigbatterie 10 weisende Lage eingenommen.
Die unterste Reihe von Luftfächerklappen 32 befindet sich gerade in einer Phase, in der die Federn 60.11 und 60.12 am stärksten zusammengezogen und der Exzenter-Schwinghebel 55.1 am meisten ausgelenkt sind. Demgemäß sind die Luftfächerklappen 32 nach rechts ausgelenkt und haben bereits den Rückweg begonnen. Wie ersichtlich, sind die verschiedenen Zugorgane und damit die Fächerklappen phasenversetzt zusammen von einem gemeinsamen Antrieb angetrieben. Dadurch gleichen sich die zunehmenden und abnehmenden Federkräfte stets aus, so daß nur die Differenzkräfte vom Antrieb aufgebracht zu werden brauchen. Das ganze System wird mittels eines relativ langsam laufenden Getriebemotors 51 in einen solchen hin- und herschwingenden Zustand versetzt, daß sich die Schwingkräfte weitgehend ausgleichen und kein ungleichmäßiger Lauf eintritt und damit die gesamte Käfigbatterie 10 in einem relativ ruhigen Zustand verbleibt. Trotz des Einsatzes relativ starker Federn bei oft viele Meter langen Zugseilen 37 lassen sich beste Antriebsergebnisse erzielen, die mit geringem Energieeinsatz zu guter Kottrocknung führen.

Bei sehr langen Käfigreihen können die Schwenkantriebe auch von jedem Ende her erfolgen und beispielsweise jeweils etwa bis zur Mitte reichen.

Nachfolgend wird ein wichtiger Teil der Beschreibung wiedergegeben:
Die Luftströmungseinrichtungen (23) von Käfigbatterien (10) für die Geflügelhaltung haben schwenkbar in Kammern (30) aufgehängte Luftfächerklappen (32) mit hin- und hergehend angetriebenen Zugseilen (37), welche diese umgebende Federn (60) aufweisen.

## Patentansprüche

1. Antrieb für Luftströmungseinrichtung (23) einer Käfigbatterie (10) für die Geflügelhaltung mit wenigstens zwei benachbart angeordneten, horizontalen Käfigreihen(11), unter denen sich jeweils eine Kotaufnahme (Kotband 20) erstreckt, der zum Trocknen des aufgefangenen Kots eine Luftströmungseinrichtung (23) zugeordnet ist, mittels der über die Kotaufnahme hinwegstreichende Luftströme erzeugt werden, wobei die Luftströmungseinrichtung (23) mehrere, über der Kotaufnahme (20) in Längsabständen verteilt angeordnete und auf Querachsen (33) gelagerte Luftfächerklappen (32) aufweist, denen zumindest gruppenweise zusammengefaßte Schwenkantriebe zum Verschwenken der Luftfächerklappen (32) zugeordnet sind, wobei die Luftfächerklappen (32) in der Mittelstellung der Schwenkantriebe im wesentlichen senkrecht ausgerichtet sind und wobei der Schwenkantrieb sich in Längsrichtung durch die Käfigbatterie erstreckende, in geringem Abstand von den Querachsen (33) der Luftfächerklappen (32), an diesen angreifende Zugorgane aufweist, die jeweils phasenversetzt zueinander hin- und hergehend und zumindest gruppenweise gemeinsam antreibbar sind,
und wobei
an jedem Zugorgan (37.1, 37.2, 37.3) mindestens eine entgegen der Richtung der Antriebskraft wirkende Feder (60) angeordnet ist.

2. Antrieb für Luftströmungseinrichtung nach Anspruch 1,
**dadurch gekennzeichnet,** daß
die Federn (60) als die Zugorgane (37.1 bis 37.3) umgebende Druckfedern ausgebildet sind.

3. Antrieb für Luftströmungseinrichtung nach Anspruch 1 oder 2,
**dadurch gekennzeichnet,** daß
wenigstens zwei Federn (60.11, 60.12; 60.21, 60.22, 60.31, 60.32) je Zugorgan (37.1, 37.2, 37.3) in unterschiedlichen Abständen vom Antrieb (51, 54, 55) angeordnet sind.

4. Antrieb für Luftströmungseinrlchtung nach wenigstens einem der übrigen Ansprüche,
**dadurch gekennzeichnet,** daß
die Federn (60) unterschiedliche Federkräfte, Durchmesser und/oder Längen aufweisen.

5. Antrieb für Luftströmungseinrichtung nach wenigstens einem der übrigen Ansprüche,
**dadurch gekennzeichnet,** daß
der Antrieb mit Hilfe von schwingend Zugkräfte aufbringenden Antriebsgliedern erfolgt.

6. Antrieb für Luftströmungseinrichtung nach Anspruch 5,
**dadurch gekennzeichnet,** daß
die Antriebsglieder mit Kurbel-Schwinghebeln (55.1 bis 55.3) ausgebildet sind.

7. Antrieb für Luftströmungseinrichtung nach Anspruch 6,
**dadurch gekennzeichnet,** daß
die Kurbel-Schwinghebel (55.1 bis 55.3) mit Exzentern (54.1 bis 54.3) oder Kurvenscheiben antreibbar sind.

8. Antrieb für Luftströmungseinrichtung nach wenigstens einem der übrigen Ansprüche,
**dadurch gekennzeichnet,** daß
die einem gemeinsamen Antriebsmotor (51) zugeordneten Antriebsglieder untereinander mit winkeleinstellbaren Kupplungen (52) verbunden sind.

9. Antrieb für Luftströmungseinrichtung nach wenigstens einem der übrigen Ansprüche,
**dadurch gekennzeichnet,** daß
ein gemeinsamer Drehantrieb, vorzugweise Getriebemotor (51) mit einer senkrecht stehenden, in mehrere, durch Kupplungen (52) verbundene Abschnitte (50.1 bis 50.4) unterteilten Antriebswelle (50) gebildet ist, auf der die Exzenterscheiben (54.1, 54.2, 54.3) befestigt sind.

10. Antrieb für Luftströmungseinrlchtung nach Anspruch 9,
**dadurch gekennzeichnet,** daß
die Exzenterscheiben (54.1 bis 54.3) mit Winkeleinstell- und Arretiermitteln auf der Antriebswelle (50) befestigbar sind.

11. Antrieb für Luftströmungseinrichtung nach wenigstens einem der übrigen Ansprüche,
**dadurch gekennzeichnet,** daß
je Käfigreihe (11) von jedem Ende bis etwa zur Mitte je ein Schwenkantrieb (37, 60, 55, 54, 50, 51) vorgesehen ist.

12. Antrieb für Lufströmungseinrichtung nach wenigstens einem der übrigen Ansprüche,
**dadurch gekennzeichnet,** daß
die Zugorgane Zugseile (37; 37.1, 37.2, 37.3) sind.

## Claims

1. A drive for an air flow device (23) of a cage battery (10) for holding poultry with at least two adjacently arranged, horizontal cage rows (11), beneath which a faeces receptacle (faeces belt 20) extends in each case, an air flow device (23) being associated with each faeces receptacle in order to dry the collected faeces and generating the air flows over the faeces receptacle, the air flow device (23) comprising a plurality of air compartment flaps (32), which are distributed at longitudinal intervals over the faeces receptacle (20), are mounted on transverse axes (33) and with which pivot drives combined at least in groups are associated for pivoting the air compartment flaps (32), the air compartment flaps (32) being substantially vertically aligned in the central position of the pivot drive and the pivot drive comprising traction members extending in the longitudinal direction through the cage battery at a short distance from the transverse axes (33) of the air compartment flaps (32) and acting upon the said flaps, the traction members being drivable together at least in groups so as to reciprocate with mutual phase displacement,
and wherein
at least one spring (60) acting against the direction of the drive force is arranged on each traction member (37.1, 37.2, 37.3).

2. A drive for an air flow device according to claim 1, characterised in that the springs (60) are constructed as compression springs surrounding the traction members (37.1 to 37.3).

3. A drive for an air flow device according to claim 1 or 2, characterised in that at least two springs (60.11, 60.12; 60.21, 60.22, 60.31, 60.32) per traction member (37.1, 37.2, 37.3) are arranged at different distances from the drive (51, 54, 55).

4. A drive for an air flow device according to at least one of the preceding claims, characterised in that the springs (60) have different spring forces, diameters and/or lengths.

5. A drive for an air flow device according to at least one of the preceding claims, characterised in that the drive is effected with the aid of drive elements generating oscillating traction forces.

6. A drive for an air flow device according to claim 5, characterised in that the drive elements are constructed with cranked rocker arms (55.1 to 55.3).

7. A drive for an air flow device according to claim 6, characterised in that the cranked rocker arms (55.1 to 55.3) are drivable by means of eccentrics (54.1 to 54.3) or cam discs.

8. A drive for an air flow device according to at least one of the preceding claims, characterised in that the drive elements associated with a common drive motor (51) are connected to one another by means of angularly adjustable couplings (52).

9. A drive for an air flow device according to at least one of the preceding claims, characterised in that a common rotary drive is formed, preferably a geared motor (51) with a vertical drive shaft (50) divided into a plurality of sections (50.1 to 50.4) connected by couplings (52), on which drive shaft (50) the eccentric discs (54.1, 54.2, 54.3) are secured.

10. A drive for an air flow device according to claim 9, characterised in that the eccentric discs (54.1 to 54.3) can be secured to the drive shaft (50) by means of angularly adjustable and lockable means.

11. A drive for an air flow device according to at least one of the preceding claims, characterised in that a respective pivot drive (37, 60, 55, 54, 50, 51) extending from each end approximately to the centre is provided per cage row (11).

12. A drive for an air flow device according to at least one of the preceding claims, characterised in that the traction members are traction cables (37; 37.1, 37.2, 37.3).

## Revendications

1. Entraînement pour un mécanisme d'aération (23) d'une batterie de cages (10) d'élevage pour volaille avec au moins deux rangées de cages (11) horizontales et voisines, au-dessous desquelles s'étend respectivement une réception de fiente (tapis roulant 20), à laquelle est associé un mécanisme d'aération (23) pour le séchage de la fiente reçue, et au moyen duquel sont produits des courants d'air passant au-dessus de la réception de fiente, le mécanisme d'aération (23) présentant plusieurs volets d'aération (32) agencés de façon répartie au-dessus de la réception de fiente (20) à des distances longitudinales et logés sur des axes transversaux (33), volets auxquels sont associés des entraînements basculants réunis du moins par groupes pour le basculement des volets d'aération (32), les volets d'aération (32) étant positionnés sensiblement verticalement dans la position centrale des entraînements basculants et l'entraînement basculant présentant des organes de traction qui s'étendent en direction longitudinale à travers la batterie de cages à une faible distance des axes transversaux (33) des volets d'aération (32) et qui attaquent ces derniers ; lesdits organes de traction pouvant être entraînés en commun en décalage de phase l'un par rapport à l'autre en va-et-vient et du moins par groupes ; et dans lequel au moins un ressort (60) agissant contre la direction de la force d'entraînement est agencé sur chaque organe de traction (37.1, 37.2, 37.3).

2. Entraînement pour mécanisme d'aération selon la revendication 1, caractérisé en ce que les ressorts (60) sont réalisés en tant que ressorts de compression qui entourent les organes de traction (37.1 à 37.3).

3. Entraînement pour mécanisme d'aération selon l'une ou l'autre des revendications 1 et 2, caractérisé en ce qu'au moins deux ressorts (60.11, 60.12 ; 60.21, 60.22, 60.31, 60.32) par organe de traction (37.1, 37.2, 37.3) sont agencés à des distances différentes de l'entraînement (51, 54, 55).

4. Entraînement pour mécanisme d'aération selon l'une au moins des revendications précédentes, caractérisé en ce que les ressorts (60) présentent des forces différentes, des diamètres différents et/ou des longueurs différentes.

5. Entraînement pour mécanisme d'aération selon l'une au moins des revendications précédentes, caractérisé en ce que l'entraînement se fait à l'aide d'organes d'entraînement qui produisent des forces de traction oscillantes.

6. Entraînement pour mécanisme d'aération selon la revendication 5, caractérisé en ce que les organes d'entraînement sont réalisés avec des leviers oscillants à manivelle (55.1 à 55.3).

7. Entraînement pour mécanisme d'aération selon la revendication 6, caractérisé en ce que les leviers oscillants à manivelle (55.1 à 55.3) peuvent être entraînés avec des excentriques (54.1 à 54.3) ou des disques à came.

8. Entraînement pour mécanisme d'aération selon l'une au moins des revendications précédentes, caractérisé en ce que les organes d'entraînement associés à un moteur d'entraînement (51) commun sont reliés mutuellement par des accouplements (52) réglables en position angulaire.

9. Entraînement pour mécanisme d'aération selon l'une au moins des revendications précédentes, caractérisé en ce qu'un entraînement rotatif commun, de préférence un moteur d'entraînement (51), est formé avec un arbre d'entraînement (50) vertical divisé en plusieurs sections (50.1 à 50.4) reliées par des accouplements (52), arbre sur lequel sont fixés les disques à excentrique (54.1, 54.2, 54.3).

10. Entraînement pour mécanisme d'aération selon la revendication 9, caractérisé en ce que les disques à excentrique (54.1 à 54.3) peuvent être fixés sur l'arbre d'entraînement (50) par des moyens de réglage angulaire et des moyens d'arrêt.

11. Entraînement pour mécanisme d'aération selon l'une au moins des revendications précédentes, caractérisé en ce que par rangée de cages (11) est prévu respectivement un entraînement basculant (37, 60, 55, 54, 50, 51) depuis chaque extrémité jusqu'à peu près au centre.

12. Entraînement pour mécanisme d'aération selon l'une au moins des revendications précédentes, caractérisé en ce que les organes de traction sont des câbles de traction (37 ; 37.1, 37.2, 37.3).
